# EUROPÄISCHE PATENTSCHRIFT

(11) **EP 0 770 049 B1**
(45) Veröffentlichungstag und Bekanntmachung des Hinweises auf die Patenterteilung: **22.09.1999**
(21) Anmeldenummer: 95925826.0
(22) Anmeldetag: 04.07.1995
(51) Int. Cl.: C07C 29/17, B01J 23/89, B01J 23/66, C07C 33/035

(54) **VERFAHREN UND KATALYSATOR ZUR SELEKTIVHYDRIERUNG VON BUTINDIOL ZU BUTENDIOL**
PROCESS AND CATALYST FOR THE SELECTIVE HYDROGENATION OF BUTINE DIOL TO BUTENE DIOL
PROCEDE ET CATALYSEUR DESTINES A L'HYDROGENATION SELECTIVE DE BUTINE DIOL EN BUTENE DIOL

(30) Priorität: 06.07.1994 DE 4423738
(43) Veröffentlichungstag der Anmeldung: 02.05.1997
(73) Patentinhaber: BASF AKTIENGESELLSCHAFT, 67056 Ludwigshafen (DE)
(72) Erfinder: IRGANG, Matthias, D-69121 Heidelberg (DE); MENGER, Volkmar, D-67434 Neustadt (DE); MIESEN, Ernest, D-67069 Ludwigshafen (DE); STOPS, Peter, D-67122 Altrip (DE); GRAF, Fritz, D-67346 Speyer (DE)
(74) Vertreter: Isenbruck, Günter, Dr.
(86) Internationale Anmeldenummer: EP9502592
(87) Internationale Veröffentlichungsnummer: WO9601242

(56) Entgegenhaltungen:
- EP-A- 0 011 439
- EP-A- 0 180 108
- EP-A- 0 184 150
- EP-A- 0 312 253
- DD-A- 246 986
- DE-A- 2 431 929
- DE-A- 2 460 078
- DATABASE WPI Week 8145 Derwent Publications Ltd., London, GB; AN 81-82217D & JP,A,56 120 687 (DAINIPPON INK & CHEMICALS) , 22.September 1981

## Beschreibung

Buten-2-diol(1,4) (im folgenden "Butendiol") wird seit langem aus Butin-2-diol(1,4) (im folgendem "Butindiol"), das großtechnisch durch die Reppe-Synthese zugänglich ist, im technischen Maßstab hergestellt. Es wird für einige wichtige Pflanzenschutzmittel, Pharmazeutika und Zwischenprodukte benötigt. Dabei ist es von großer Wichtigkeit, ein möglichst sauberes Hydrierprodukt zu erhalten, da Produktverluste vermieden werden müssen und die destillative Abtrennung von nichthydriertem Butindiol und von durch Überhydrierung entstandenem Butandiol nur mit großem technischen Aufwand möglich ist.

Die katalytische Hydrierung von Butindiol zu Butendiol wird in der Regel diskontinuierlich mit einem Suspensionskatalysator durchgeführt. Dabei wird bei Temperaturen von 30 - 150°C und einem Druck zwischen 1 und 20 bar Wasserstoff in einen Rührbehälter, der die Butindiol-Lösung und den Katalysator enthält, eingespeist. Nach Aufnahme der stöchiometrischen Wasserstoffmenge wird die Reaktion beendet. Für die Katalysatoren, insbesondere für Palladium-Katalysatoren, sind zahlreiche Vorschläge gemacht und zum Teil auch technisch realisiert worden.

Zur Erzielung ausreichender Selektivität wurden Pd/BaSO₄-Katalysatoren mit Chinolinzusatz (DE 1 115 238), oder Pd/Al₂O₃-Katalysatoren mit Kohlenmonoxidzusatz (DE 2 619 660) oder Pd/Cu-bzw. Pd/Al₂O₃-Katalysatoren mit Kupferacetat-Zusatz (GB 832 141) vorgeschlagen.

Jedoch sind lösliche Zusätze in der Handhabung umständlich und in der Aufarbeitung störend. Eine CO-Dosierung, wie in DE 2619 660 beschrieben, erfordert zusätzlichen technischen Aufwand und soll daher ebenfalls vermieden werden.

Gute Ergebnisse wurden nach Patentangaben auch mit folgenden Katalysatoren erzielt:

| | |
|---|---|
| 5% Pd/BaSO₄ | DE 2 605 241 |
| 5% Pd/Al₂O₃ dotiert mit Bleiacetat | DE 2 818 260 |
| 5% Pd/BaSO₄ mit Kupfernitratzusatz | DD 246 986 |

Trotz des Einsatzes hochprozentiger Katalysatoren (5% Pd) waren für diese Hydrierungen lange Hydrierzeiten erforderlich.

DE 2 431 929 beschreibt Katalysatoren mit nur 0.5% Pd auf Al₂O₃ mit Dotierung durch Zink, Cadmium, Bismut und Tellur. Die Katalysatoren zeichnen sich durch gute Selektivität aus, haben jedoch den Nachteil, cancerogene bzw. toxische Bestandteile zu enthalten.

Eine besondere Schwierigkeit bildet die Verwendung von Roh-Butindiol, das ohne Vorreinigung in der Selektivhydrierung eingesetzt werden soll. Dieses Material mit einem pH-Wert von ca. 5 enthält Methanol, Formaldehyd, Ameisensäure und Propargylalkohol. Ferner bringt es auch Katalysatorbestandteile, wie Cu und Bi aus der Butindiolsynthese mit. Von dem Katalysator wird also auch weitgehende Toleranz gegenüber diesen Rohstoffbestandteilen gefordert, obwohl bekannt ist, daß sowohl Zusätze von Kupfer (vgl. DD 246 986) als auch von Bismut (vgl. DE 2 431 929) die Hydrierung beeinflussen.

Trotz des umfangreichen Standes der Technik besteht der Wunsch, das Verfahren zur Selektivhydrierung von Butindiol und den hierfür verwendbaren Katalysator weiter zu verbessern. Dabei bestehen insbesondere folgende Aufgaben bzw. Teilaufgaben, deren Lösung - einzeln oder in Kombination - die Erfindung austrebt:
1. Bei der Herstellung des Katalysators sollen keine toxischen oder cancerogenen Substanzen, wie z.B. Tellur- oder Cadmiumverbindungen eingesetzt werden müssen.
2. Eine möglichst hohe Selektivität soll erreicht werden, um Produktverluste insbesondere durch Überhydrierung und Acetalbildung zu minimieren.
3. Eine hohe Katalysatoraktivität soll erreicht werden, um die Katalysatoreinsatzmenge bezogen auf die umzusetzende Butindiolmenge zu senken.
4. Eine problemlose Handhabung des Katalysators, insbesondere gute Filtrierbarkeit soll erzielt werden.
5. Die Selektivhydrierung soll auch unter Verwendung von Rohbutindiol (insbesondere eines solchen mit pH = 5) durchführbar sein.
6. Die Selektivhydrierung soll auch bei Vorhandensein einer oder mehrerer folgenden Verunreinigungen durchführbar sein: Methanol, Formaldehyd, Ameisensäure, Propargylalkohol bzw. Cu und/oder Bi, die z.B. aus der Butindiolsynthese stammen können.
7. Toleranz des Katalysators gegenüber Rohstoffbestandteilen.

Diese Aufgabe bzw. Teilaufgaben der Erfindung werden mittels Verfahren und Katalysatoren gelöst, wie sie in den Ansprüchen definiert sind. Bevorzugte Ausführungsformen der Erfindung ergeben sich aus den Unteransprüchen sowie der folgenden Beschreibung und den Beispielen. Der erfindungsgemäß anwendbare Katalysator enthält neben Palladium und Zink auch Kupfer oder Silber, ist aber zweckmäßigerweise frei von Cadmium. Vorzugsweise besteht der Katalysator hinsichtlich seiner aktiven Bestandteile aus diesen Elementen (Pd + Zn + [Cu und/oder Ag]) und enthält keine weiteren aktiven Bestandteile. Der bevorzugte Katalysator ist ein Palladium-Trägerkatalysator mit den genannten aktiven Bestandteilen.

Alle Mengenangaben zu Metalloxyden hierin sind so zu verstehen, daß es sich dabei um Mengenangaben, zu dem Metall berechnet als das betreffende Oxid, handelt. Die tatsächliche Struktur kann von der angegebenen abweichen.

Erfindungsgemäß wurde ein Verfahren gefunden zur Herstellung von Buten-2-diol(1,4) durch selektive Hydrierung von Butindiol in Anwesenheit eines Palladiumkatalysators, das dadurch gekennzeichnet ist, daß der Katalysator neben Palladium die Elemente Zink und Kupfer oder Zink und Silber oder Zink und Kupfer und Silber enthält.

Die erfindungsgemäß im Verfahren zur Hydrierung von Butin-1,4-diol zu Buten-1,4-diol anwendbaren Trägerkatalysatoren enthalten in ihrer katalytisch aktiven Masse im allgemeinen 0,1 bis 7 Gew.-% Palladium, vorzugsweise 0,1 bis 4 Gew.-% Palladium, jeweils berechnet als Pd und bezogen auf das Gesamtgewicht des Katalysators. Als weitere katalytisch aktive Elemente enthält der erfindungsgemäß anzuwendende Katalysator zusätzlich zum Palladium noch die Elemente Zink und Kupfer oder Zink und Silber oder Zink und Kupfer und Silber.

Erfindungsgemäß anwendbare Katalysatoren, die in ihrer katalytisch aktiven Masse aus den katalytisch aktiven Komponenten Palladium, Zink und Kupfer bestehen, enthalten außer Palladium in den vorgenannten Mengen, die beiden anderen Elemente in Mengen, die einem Palladium : Zink-Atomverhältnis von im allgemeinen 10 : 1 bis 1 : 4 und einem Zink : Kupfer-Atomverhältnis von im allgemeinen 5 : 1 bis 1 : 2 entsprechen.

Erfindungsgemäß anwendbare Katalysatoren, die in ihrer katalytisch aktiven Masse aus den katalytisch aktiven Komponenten Palladium, Zink und Silber bestehen, enthalten außer Palladium in den vorgenannten Mengen, die Elemente Zink und Silber in Mengen, die einem Palladium : Zink-Atomverhältnis von im allgemeinen 10 : 1 bis 1 : 4 und einem Zink : Silber-Atomverhältnis von im allgemeinen 5 : 1 bis 1 : 2 entsprechen.

Erfindungsgemäß anwendbare Katalysatoren, die in ihrer katalytisch aktiven Masse aus den katalytisch aktiven Komponenten Palladium, Zink, Kupfer und Silber bestehen, enthalten außer Palladium in den vorgenannten Mengen, die Elemente Zink, Kupfer und Silber in Mengen, die einem Pallaldium : Zink-Atomverhältnis von im allgemeinen 10 : 1 bis 1 : 4, einem Zink : Kupfer-Atomverhältnis von im allgemeinen 5 : 1 bis 1 : 2 und einem Zink : Silber-Atomverhältnis von im allgemeinen 5 : 1 bis 1 : 2 entsprechen.

Die erfindungsgemäß anwendbaren Katalysatoren sind Trägerkatalysatoren. Als Träger werden vorzugsweise Trägermaterialien geringer Acidität oder basische Träger verwendet. Beispiele für vorteilhaft anwendbare Trägermaterialien sind Aluminiumoxide, Calciumcarbonat, Magnesiumoxid, Spinell (MgAl₂O₄), Bariumsulfat, Titandioxide oder Zirkoniumdioxid. Es können auch Gemische dieser Trägermaterialien zur Herstellung der erfindungsgemäß anwendbaren Katalysatoren verwendet werden. Besonders bevorzugt als Trägermaterial is Aluminiumoxid, insbesondere δ (Delta)-Aluminiumoxid, das nach Ullmanns Encyklopädie der technischen Chemie, 4. Auf., Band 7, S. 298-299, Verlag Chemie, Weinheim 1974 hergestellt werden kann. Als besonders geeignetes δ-Aluminiumoxid-Trägermaterial wurde δ-Aluminiumoxid mit einer BET-Oberfläche von 100 bis 130 m²/g (bestimmt nach C.N. Satterfield: Heterogeneous Catalysis in Practice, S. 102-105, New York 1980) und einer Korngröße von 100 bis 200 µm gefunden, das sich durch sehr gute Absetz- und Filtrationseigenschaften auszeichnet und bei dessen Verwendung als Trägermaterial nur eine geringe Acetal- und Polymerbildung beobachtet wird. Im allgemeinen haben die verwendeten Trägermaterialien jedoch eine BET-Oberfläche von 5 bis 200 m²/g, eine Porosität von 0,1 bis 1 ml/g, bestimmt durch Wasserabsorption und mittlere Partikelgrößen von 20 bis 150 µm mit einer maximalen Partikelgröße bis zu 300 µm.

Als besonders vorteilhaft erwiesen sich solche Katalysatoren, die durch Tränkung des Trägermaterials mit einer Lösung der katalytisch aktiven Katalysatorkomponenten hergestellt worden sind. Die Imprägnierung des Trägers kann dabei gleichzeitig durch Tränken mit einer Mischlösung der wasserlöslichen Salze der katalytisch aktiven Komponenten, vorzugsweise mit einer Lösung von deren Nitraten oder Acetaten, oder durch sequentielle Tränkung mit Lösungen jeweils eines dieser Salze, erfolgen, wobei zweckmäßigerweise nach den einzelnen Tränkschritten der imprägnierte Träger getrocknet wird. Die Tränkung kann durch Behandeln des Trägermaterials mit einer überstehenden Lösung dieser Salze bewerkstelligt werden, besonders vorteilhaft erfolgt sie durch Zugabe einer Mischlösung zum Träger in einer rotierenden Trommel, wobei vorteilhaft eine Menge an Lösung eingesetzt wird, die dem Porenvolumen des Trägers entspricht. Nach Trocknung und gegebenenfalls Calcination, in der Regel bei 300 bis 600°C, vorzugsweise bei 400 bis 550°C, kann der Katalysator im erfindungsgemäßen Verfahren eingesetzt werden. Die Katalysatoren können vor ihrer Anwendung im erfindungsgemäßen Verfahren, beispielsweise durch Behandlung mit Wasserstoff oder anderen Reduktionsmitteln, wie Hydrazin, aktiviert werden, in der Regel ist dies aber nicht erforderlich, da diese Katalysatoren vorteilhaft in situ in der Reaktionsmischung reduziert und aktiviert werden können.

Es wurde überraschenderweise gefunden, daß mit Kupfer und Zink dotierte Palladium auf Aluminiumoxid-Katalysatoren eine deutlich höhere Selektivität im erfindungsgemäßen Verfahren haben, als ein nur Palladium und Kupfer enthaltender Katalysator. Hierdurch konnte die Butendiolausbeute deutlich gesteigert werden. Insbesondere konnte mit diesen Pd/Zn/Cu-Katalysatoren im erfindungsgemäßen Verfahren die Summe der Nebenprodukte Butindiol, Butandiol und Acetale auf ein Niveau gesenkt werden, das unterhalb desjenigen der Pd/Zn/Al₂O₃- bzw. Pd/Zn/Cd/Al₂O₃-Katalysatoren des Standes der Technik liegt.

Als weitere sehr selektive Katalysatoren erwiesen sich im erfindungsgemäßen Verfahren mit Silber und Zink dotierte Palladium auf Aluminiumoxid-Katalysatoren. Durch den Einsatz basischer Träger, wie Calciumcarbonat oder Magnesiumoxid, kann noch eine weitere Steigerung der Ausbeute im erfindunggemäßen Verfahren erzielt werden, wobei die Menge der gebildeten Acetalnebenprodukte weiter reduziert wird.

Mit diesen Katalysatoren wurden die dem erfindungsgemäßen Verfahren zugrundeliegenden Aufgaben gelöst. Insbesondere ermöglicht die Ver-wendung dieser Katalysatoren die Erzielung besonders hoher Raum-Zeit-Ausbeuten und die Minimierung der Bildung von Nebenprodukten. Darüber hinaus sind sie im Hinblick auf Belange des Umweltschutzes und der Arbeitsplatzsicherheit problemlos handhabbar.

Die Selektivhydrierung von Butindiol zu Butendiol kann mittels der erfindungsgemäß anzuwendenden Katalysatoren nach an sich herkömmlichen Hydrierverfahrenstechniken vorgenommen werden. Vorzugsweise werden dabei die Katalysatoren in suspendierter Form im Reaktionsgemisch eingesetzt. Die Hydrierung kann bei Atmosphärendruck oder unter erhöhtem Druck durchgeführt werden. Im allgemeinen werden ein Druck von 1 bis 20 bar, vorzugsweise von 1 bis 10 bar und Temperaturen von im allgemeinen 20 bis 150°C, vorzugsweise von 50 bis 120°C, angewandt.

Als Ausgangsmaterial kann reines Butindiol oder dessen Lösungen in einem geeigneten Lösungsmittel, z.B. Wasser, eingesetzt werden, vorzugsweise wird im erfindungsgemäßen Verfahren eine Rohbutindiollösung eingesetzt, wie sie z.B. bei der Butindiolherstellung nach Reppe anfällt. Dieses Rohbutindiol enhält im allgemeinen ca. 50 Gew.-% Wasser und 1,5 bis 2,5 Gew.-% Verunreinigungen aus der Reppe-Synthese. Obwohl diese Verunreinigungen im Zuge der Hydrierung zur Bildung von Nebenprodukten und teerartigen Rückständen neigen, kann mit Hilfe der erfindungsgemäß anzuwendenden Katalysatoren diese Nebenprodukt- und Rückstandsbildung minimiert werden.

Der Wasserstoff kann dem Hydrierreaktor, vorzugsweise einem Rührbehälter mit Begasungsrührer, in bezüglich des Butindiols stöchiometrischer oder überschüssiger Menge zugeführt werden, bevorzugt wird der Wasserstoff im stöchiometrischer Menge eingesetzt.

Zur Aufarbeitung der Reaktionsmischung wird diese im allgemeinen, zweckmäßigerweise nach vorheriger Abtrennung des Katalysators, beispielsweise durch Filtration oder Zentrifugation, destilliert.

Dabei werden zweckmäßigerweise zunächst Wasser und ein Vorlauf, der vor allem Allylalkohol enthält, entfernt. Anschließend wird zweckmäßigerweise das Butendiol, zusammen mit den Nebenprodukten Butandiol, Butindiol und Acetalen vom hochsiedenden Rückstand destillativ abgetrennt, wonach das Butendiol in einer Reindestillation isoliert werden kann.

Das erfindungsgemäße Verfahren kann sowohl diskontinuierlich, z.B. in Rührautoklaven, oder kontinuierlich, z.B. in Rührkesselkaskaden, ausgeführt werden. Vorzugsweise wird das Verfahren diskontinuierlich (chargenweise) durchgeführt.

### Beispiele

Die Gew.-% Angaben in den Beispielen beziehen sich auf den Gesamtkatalysator als 100 Gew.-%.
Katalysator A (Vergleichsbeispiel).
   In einer rotierenden Trommel wurden 5 kg Delta-Al₂O₃ mit einer Korngröße von 100 - 200 µm vorgelegt und mit einer Mischlösung von Palladiumnitrat und Kupfernitrat besprüht. Die Lösungsmenge wurde so berechnet, daß die Porenfüllung des Trägers erreicht wurde, für 5 kg des angegebenen Trägers wurden ca. 2500 ml Lösung benötigt. Nachdem die Lösung vollständig aufgenommen wurde, wurde der Katalysator bei 120°C getrocknet und bei 500°C calciniert. Seine Zusammensetzung lautete:
   0.5 Gew.% Pd
   0.25 Gew.% CuO
   Rest Al₂O₃
Katalysator B (Vergleichsbeispiel).
   Man verfuhr wie bei der Herstellung von Katalysator A und verwendete zur Tränkung eine Mischlösung aus Palladiumnitrat und Zinknitrat. Die Katalysatorzusammensetzung lautete:
   0.5 Gew.% Pd
   0.25 Gew.% ZnO
   Rest Al₂O₃
Katalysator C (Vergleichsbeispiel).
   Man verfuhr wie bei der Herstellung von Katalysator A und verwendete zur Tränkung eine Mischlösung aus Palladiumnitrat, Cadmiumnitrat und Zinknitrat. Die Katalysatorzusammensetzung lautete:
   0.5 Gew.% Pd
   0.11 Gew.% CdO
   0.12 Gew.% ZnO
   Rest Al₂O₃
Katalysator D
   Man verfuhr wie bei der Herstellung von Katalysator A und verwendete zur Tränkung eine Mischlösung aus Palladiumnitrat, Kupfernitrat und Zinknitrat. Die Katalysatorzusammensetzung lautete:
   0.5 Gew.% Pd
   0.12 Gew.% CuO
   0.12 Gew.% ZnO
   Rest Al₂O₃
Katalysator E.
   Man verfuhr wie bei der Herstellung von Katalysator A und verwendet zur Tränkung eine Mischlösung aus Palladiumnitrat, Silbernitrat und Zinknitrat. Die Katalysatorzusammensetzung lautete:
   0.5 Gew.% Pd
   0.11 Gew.% Ag₂O
   0.12 Gew.% ZnO
   Rest Al₂O₃
Katalysator F.
   Man verfuhr wie bei der Herstellung von Katalysator D, legte jedoch für die Tränkung pulverförmiges, gefälltes Calciumcarbonat vor. Die erforderliche Lösungsmenge betrug ca. 2500 ml für 5 kg Träger. Die Katalysatorzusammensetzung lautete:
   0.5 Gew.% Pd
   0.11 Gew.% CuO
   0.11 Gew.% ZnO
   Rest CaCO₃
Katalysator G.
   Man verfuhr wie bei der Herstellung von Katalysator D, legte jedoch für die Tränkung gefälltes Magnesiumoxid vor, das durch Kompaktierung und Aussiebung eine Korngröße von 100 - 300 µm erhalten hatte. Die Katalysatorzusammensetzung lautete:
   0.5 Gew.% Pd
   0.10 Gew.% CuO
   0.10 Gew.% ZnO
   Rest MgO

**Tab. I**

| Katalysator-Prüfung (Kleinautoklav) | | | |
|---|---|---|---|
| Katalysator | Aktivkomponenten | Träger | Hydrierzeit min. |
| A Vergleich | Pd/Cu | Al₂O₃ | 87 |
| B Vergleich | Pd/Zn | Al₂O₃ | 62 |
| C Vergleich | Pd/Zn/Cd | Al₂O₃ | 86 |
| D Erfindung | Pd/Zn/Cu | Al₂O₃ | 48 |
| E Erfindung | Pd/Zn/Ag | Al₂O₃ | 69 |
| F Erfindung | Pd/Zn/Cu | CaCO₃ | 59 |
| G Erfindung | Pd/Zn/Cu | MgO | 114 |

| | Nebenprodukte im Hydrieraustrag | | | |
|---|---|---|---|---|
| Katalysator | Butandiol | "Acetal" Fl.% | Butindiol Fl.% | Summe Fl.% |
| A Vergleich | 1.30 | 0.3 | 1.60 | 3.2 |
| B Vergleich | 3.1 | 0.7 | 0.1 | 3.9 |
| C Vergleich | 1.6 | 0.35 | 1.3 | 3.25 |
| D Erfindung | 1.3 | 0.3 | 0.8 | 2.4 |
| E Erfindung | 2.0 | 0.5 | 0.1 | 2.6 |
| F Erfindung | 0.9 | 0.14 | 0.1 | 1.14 |
| G Erfindung | 2.1 | 0.2 | 0 | 2.3 |

### Katalysator-Prüfung

Die Hydrierversuche wurden in einem Kleinautoklav mit Magnethubrührer bei 100°C und 18 bar durchgeführt. Als Edukt wurden 125 ml Rohbutindiol-Lösung, der 150 mg Katalysator zugesetzt wurden, verwendet. Der Wasserstoffverbrauch wurde durch die Druckverminderung im Autoklav kontrolliert; der verbrauchte Wasserstoff wurde periodisch ergänzt. Wegen der schwierigen Erkennung des Endpunktes bei der Selektivhydrierung zum Butendiol wurden generell etwas höhere Nebenproduktwerte als in der Produktionsanlage, die eine bessere Endpunktserkennung gestattet, gefunden.

Das Hydrierprodukt wurde gaschromatographisch analysiert; die Ergebnisse wurden in Flächenprozenten (Fl.%) angegeben. Die Vorlaufmengen waren vom Katalysator unabhängig und durch die Qualität des Rohbutindiols gegeben.

Die Daten für das Hydrierprodukt sind in Tab. I zusammengefaßt. Die Rückstandsmengen wurden nur für die Katalysatoren B, C und D bestimmt. Sie betragen jeweils bezogen auf 100 g Hydrierprodukt:
für Katalysator B 17.5 g
für Katalysator C 10.8 g
für Katalysator D 12.9 g.

Die Ergebnisse sind Durchschnittswerte aus fünf Versuchen.

### Hydrierergebnisse aus Produktionsanlage

Im technischen Maßstab wurden die Katalysatoren B, C und D in einer Langzeiterprobung getestet. Die Butindiolhydrierung erfolgte diskontinuierlich in Suspensionsfahrweise. Es wurde pro m³ Rohbutindiol 1 kg Katalysator eingesetzt.

Der Nebenproduktgehalt, angegeben in Flächenprozent der gaschromatographischen Auswertung, wurde wie folgt ermittelt:

| Katalysator | Butandiol | "Acetal" | Butindiol | Summe (an+ac+in) |
|---|---|---|---|---|
| B | 1.34% | 0.47% | 0.35% | 2.16% |
| C | 0.94% | 0.37% | 0.53% | 1.84% |
| D | 0.83% | 0.33% | 0.29% | 1.45% |

Diese Daten belegen die Vorteile, die sich durch die Verwendung des erfindungsgemäßen Katalysators D ergeben.

## Patentansprüche

1. Verfahren zur Herstellung von Buten-2-diol(1,4) durch Hydrierung von Butindiol in Anwesenheit eines Palladiumkatalysators, dadurch gekennzeichnet, daß man einen Katalysator verwendet, der neben Palladium die Elemente Zink und Kupfer oder Zink und Silber oder Zink und Kupfer und Silber enthält.

2. Verfahren nach Anspruch 1, dadurch gekennzeichnet, daß man einen Palladiumträgerkatalysator mit einem Palladiumgehalt von 0,1 bis 7 Gew.-% berechnet als Pd und bezogen auf das Gesamtgewicht des Katalysators, verwendet.

3. Verfahren nach den Ansprüchen 1 und 2, dadurch gekennzeichnet, daß man einen Palladiumträgerkatalysator verwendet, in dem das Atomverhältnis der katalytisch aktiven Elemente
Palladium : Zink 10 :1 bis 1 : 4
und
Zink : Kupfer 5 : 1 bis 1 : 2 beträgt.

4. Verfahren nach den Ansprüchen 1 und 2, dadurch gekennzeichnet, daß man einen Palladiumträgerkatalysator verwendet, in dem das Atomverhältnis der katalytisch aktiven Elemente
Palladium : Zink 10 : 1 bis 1 : 4
und
Zink : Silber 5 : 1 bis 1 : 2 beträgt.

5. Verfahren nach den Ansprüchen 1 und 2, dadurch gekennzeichnet, daß man einen Palladiumkatalysator verwendet, in dem das Atomverhältnis der katalytisch aktiven Elemente
Palladium : Zink 10 : 1 bis 1 : 4,
Zink : Kupfer 5 : 1 bis 1 : 2 und
Zink : Silber 5 : 1 bis 1 : 2 beträgt.

6. Verfahren nach den Ansprüchen 1 bis 5, dadurch gekennzeichnet, daß man einen Palladiumträgerkatalysator verwendet, bei dem die katalytisch aktiven Elemente auf einem basischen Trägermaterial oder einem Trägermaterial mit geringer Acidität aufgebracht sind.

7. Verfahren nach den Ansprüchen 1 bis 6, dadurch gekennzeichnet, daß man einen Palladiumträgerkatalysator auf einem Träger aus Aluminiumoxid, Calciumcarbonat, Magnesiumoxid, Spinell, Bariumsulfat, Titandioxid, Zirkoniumdioxid oder Gemischen dieser Trägermaterialien verwendet.

8. Verfahren nach den Ansprüchen 1 bis 7, dadurch gekennzeichnet, daß man einen Palladiumträgerkatalysator auf einem Träger aus δ-Aluminiumoxid verwendet.

9. Verfahren nach den Ansprüchen 1 bis 8, dadurch gekennzeichnet, daß man die Umsetzung bei Temperaturen von 20 bis 150°C und bei einem Druck von 1 bis 20 bar durchführt.

10. Verfahren nach den Ansprüchen 1 bis 9, dadurch gekennzeichnet, daß man als Ausgangsmaterial eine Lösung von Rohbutindiol aus der Reppe-Synthese einsetzt.

## Claims

1. A process for the preparation of 2-butene-1,4-diol by hydrogenation of butynediol in the presence of a palladium catalyst, wherein a catalyst is used which, in addition to palladium, contains the elements zinc and copper or zinc and silver or zinc and copper and silver.

2. A process as claimed in claim 1, wherein a palladium supported catalyst having a palladium content of from 0.1 to 7% by weight, calculated as Pd and based on the total weight of the catalyst, is used.

3. A process as claimed in claim 1 or 2, wherein a palladium supported catalyst is used in which the atomic ratio between the catalytically active elements is
palladium:zinc 10:1 to 1:4 and
zinc:copper 5:1 to 1:2.

4. A process as claimed in claim 1 or 2, wherein a palladium supported catalyst is used in which the atomic ratio between the catalytically active elements is
palladium:zinc 10:1 to 1:4 and
zinc:silver 5:1 to 1:2.

5. A process as claimed in claim 1 or 2, wherein a palladium supported catalyst is used in which the atomic ratio between the catalytically active elements is
palladium:zinc 10:1 to 1:4,
zinc:copper 5:1 to 1:2 and
zinc:silver 5:1 to 1:2.

6. A process as claimed in any of claims 1 to 5, wherein a palladium supported catalyst is used in which the catalytically active elements have been applied to a basic or low-acidity support material.

7. A process as claimed in any of claims 1 to 6, wherein a palladium supported catalyst on a support of aluminum oxide, calcium carbonate, magnesium oxide, spinel, barium sulfate, titanium dioxide, zirconium dioxide or mixtures thereof is used.

8. A process as claimed in any of claims 1 to 7, wherein a palladium supported catalyst on a support of 6-aluminum oxide is used.

9. A process as claimed in any of claims 1 to 8, wherein the reaction is carried out at from 20 to 150°C and from 1 to 20 bar.

10. A process as claimed in any of claims 1 to 9, wherein the starting material is a solution of crude butynediol from the Reppe synthesis.

## Revendications

1. Procédé de préparation de butène-2-diol(1,4) par hydrogénation de butynediol en présence d'un catalyseur au palladium, caractérisé en ce que l'on utilise un catalyseur qui, outre du palladium, contient également les éléments zinc et cuivre, ou zinc et argent, ou zinc et cuivre et argent.

2. Procédé selon la revendication 1, caractérisé en ce que l'on utilise un catalyseur support de palladium d'une teneur en palladium de 0,1 à 7% en poids, calculée en tant que Pd et ramenée au poids total du catalyseur.

3. Procédé selon les revendications 1 et 2, caractérisé en ce que l'on utilise un catalyseur support de palladium dans lequel les rapports atomiques des éléments catalytiquement actifs sont respectivement
Palladium:zinc 10:1 à 1:4
et
Zinc : cuivre 5:1 à 1:2.

4. Procédé selon les revendications 1 et 2, caractérisé en ce que l'on utilise un catalyseur support de palladium dans lequel les rapports atomiques des éléments catalytiquement actifs sont respectivement
Palladium:zinc 10:1 à 1:4
et
Zinc : argent 5:1 à 1:2.

5. Procédé selon les revendications 1 et 2, caractérisé en ce que l'on utilise un catalyseur au palladium dans lequel les rapports atomiques des éléments catalytiquement actifs sont respectivement
Palladium:zinc 10:1 à 1:4,
Zinc:cuivre 5:1 à 1:2 et
Zinc : argent 5:1 à 1:2.

6. Procédé selon les revendications 1 à 5, caractérisé en ce que l'on utilise un catalyseur support de palladium dans lequel les éléments catalytiquement actifs sont déposés sur un matériau de support basique ou un matériau de support légèrement acide.

7. Procédé selon les revendications 1 à 5, caractérisé en ce que l'on utilise un catalyseur support de palladium sur un support constitué d'oxyde d'aluminium, de carbonate de calcium, d'oxyde de magnésium, de spinelle, de sulfate de baryum, de dioxyde de titane, de dioxyde de zirconium ou de mélanges de ces matériaux de support.

8. Procédé selon les revendications 1 à 7, caractérisé en ce que l'on utilise un catalyseur support de palladium sur un support de 6-oxyde d'aluminium.

9. Procédé selon les revendications 1 à 8, caractérisé en ce que l'on entreprend la conversion à des températures de 20 à 150°C et sous une pression de 1 à 20 bar.

10. Procédé selon les revendications 1 à 9, caractérisé en ce que l'on utilise comme matériau de départ une solution de butyne diol brute provenant de la synthèse de Reppe.
